Europäisches Patentamt

European Patent Office

Office européen des brevets

(19)

(11) Publication number: **0 040 445**
**B1**

(12) **EUROPEAN PATENT SPECIFICATION**

(45) Date of publication of patent specification: **18.04.84**

(51) Int. Cl.³: **E 21 B 43/00, C 09 K 7/00**

(21) Application number: **81200479.4**

(22) Date of filing: **06.05.81**

(54) Fluid displacement with heteropolysaccharide solutions, and the microbial production of heteropolysaccharides.

(30) Priority: **21.05.80 GB 8016832**

(43) Date of publication of application:
**25.11.81 Bulletin 81/47**

(45) Publication of the grant of the patent:
**18.04.84 Bulletin 84/16**

(84) Designated Contracting States:
**CH DE GB LI NL**

(56) References cited:
**EP - A - 0 001 895**
**GB - A - 1 539 064**
**US - A - 4 128 482**
**US - A - 4 212 748**
**US - A - 4 256 590**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Cripps, Roger Edward**
**Carel van Bylandtlaan 30**
**NL-2596 HR The Hague (NL)**
Inventor: **Ruffell, Richard Norman**
**"Rosherville" The Green**
**Bearsted Nr. Maidstone Kent (GB)**
Inventor: **Sturman, Anthony John**
**9 Everard Way**
**Faversham Kent (GB)**

(74) Representative: **Hunter, Keith Roger Ian et al,**
**4 York Road**
**London SE1 7NA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# 0 040 445

Fluid displacement with heteropolysaccharide solutions, and the microbial production of heteropolysaccharides

This invention relates to a process for displacing a fluid through a permeable subsurface formation communicating with a well, by injecting into the formation via said well an aqueous solution of a microbially produced polysaccharide. The invention also relates to a microbial production of certain polysaccharides suitable for use in such displacement process.

A permeable subsurface formation, which is referred to herein by the expression "oil-bearing formation" or "oil reservoir", is a permeable rock formation having the pores thereof at least partly filled with a liquid and/or gaseous mixture of hydrocarbons. The formation is bounded at its upper side by a layer of impervious rock through which liquid and gas cannot pass. Originally the pores in the formation were filled with water. Oil and gas that were formed in adjacent source rocks gradually seeped and bubbled through the pores until they were trapped by the impermeable rock barrier.

In order to recover the valuable fluids from a subsurface formation, a well or a plurality of wells is drilled from the surface into the formation. In the course of the recovery process, several treatments have to be carried out in the well(s) and/or the formation, wherein a fluid—this term including gas(es) and/or liquid(s)—present in the well(s) and/or the formation is displaced by a viscous aqueous solution. Such treatments include, inter alia, completion or work-over operations, enhanced recovery operations, and fracturing operations.

Completion or work-over operations include the cleaning of a well by removing the liquid originally present therein. This liquid is then displaced by a viscous aqueous solution, which is circulated at least once through the well (via a conduit extending in the well and the annulas around the conduit). When passing the circulating viscous solution more than once through the well, the solution may be filtered at the surface to remove solid particles therefrom that are swept from the well by the solution. Since the viscous solution is in contact with the face of the formation at the level where the formation and the well communicate, the properties of the viscous solution should be such that the fluid communication between the well and the formation is not impaired by that part of the viscous solution that enters the formation pore space during the treatment. In order to increase the density of the fluid and/or to prevent shale swelling (which would result in a reduction of injectivity of the formation) a brine solution is often used. (Brine solutions are aqueous solutions of salts such as NaCl, $CaCl_2$, $MgCl_2$, $CaBr_2$ and the like or mixtures thereof).

Polysaccharides, and in particular microbial polysaccharides, are often applied as a viscosifier to increase the viscosity of the solution that is used for displacing the liquid originally present in the well in carrying out completion or work-over operations in the well. For the reasons set out above, the viscous solution should be salt-resistant (i.e. its physical, and in particular its rheological, properties should remain substantially unaffected by the presence of ionic salts) and should be filterable by a filter without excessively plugging the filter. However, those microbial polysaccharides which are at present available generally fail to satisfy these requirements completely.

The same problems exist when using an aqueous viscous solution in a fracturing process, in which a fracture is formed in the formation part adjoining a well by injecting a fracturing liquid into the formation pore space at a pressure above the formation breakdown pressure. The fracturing fluid is propelled through the well and the formation by a viscous aqueous solution. The fracturing liquid enters the pore space at a pressure higher than the fracturing pressure, thereby causing a fracture in the formation. The fracturing liquid is propelled deeper into the formation by the viscous displacement fluid which is also employed to carry into the fracture propping agents that will keep the fracture walls at some distance from one another when the pressure in the fracture is released. The viscosifier used for obtaining the desired viscosity of the displacement liquid should be salt-resistant (since the liquid is preferably a brine solution to prevent swelling of clay or shale present in the fracture walls). Further, the viscosifier should not plug the entrances to the pore space of the formation when the displacement fluid is in contact with the fracture walls. As noted above with reference to the liquid displacement process forming part of a well completion or work-over process, the currently available microbial polysaccharides do not completely satisfy those requirements.

The same problems also exist in enhanced oil recovery processes wherein the fluids present in the formation pore space are displaced to a production well or a plurality of production wells that are in communication with such formation pore space. Such displacement processes are required in oil recovery operations when the natural forces for driving the oil to production wells are insufficient to displace the oil (that is often of a high viscosity) at an economical rate. One solution is to pump gas or water from the surface into the reservoir through injection wells located at some distance from the production wells. Because gas is valuable, and water is as a rule more efficient at displacing oil than is gas, injection of water has become a conventional agent used in enhanced oil recovery processes.

When water is injected in the formation for displacing oil in the reservoir to the production wells, tongues and fingers of water will develop that advance below and through, respectively the body of oil present in the pore space of the formation and ahead of the main water mass. The drive water thereby by-passes part of the oil, which results in a decrease in the sweep effect of the water. Moreover, substantial amounts of water will be produced with the oil and have to be separated therefrom. The

2

problem is more severe in the recovery of viscous oils, and one technique for alleviating this problem is to increase the viscosity of the drive water by the dissolution of suitable chemicals. Certain water-soluble polymers have been proposed for this application, but these often fail to retain their full potential effectiveness under the severe physical conditions encountered in the oil-bearing formation. In particular, in addition to having the essential inherent viscosity in aqueous solution, the polymer should be adequately shear resistant and should not cause plugging of the pores in the formation, and these qualities should be retained in the conditions of salinity and temperature which prevail in oil reservoirs.

It has now been discovered that certain microbially produced polysaccharides have properties well-suited to their use in displacing liquids through wells and/or permeable subsurface formations communicating with these wells, which displacement processes may be included, inter alia, in well completion and work-over operations, fracturing methods and enhanced oil recovery techniques.

The present invention therefore provides a process for displacing a fluid through a well and/or a permeable subsurface formation by injecting into the well an aqueous solution, characterised in that the solution contains as viscosifier a heteropolysaccharide comprising glucose and, for each 7 moles of glucose, 0.9 to 1.2 moles of galactose and 0.65 to 1.1 moles of pyruvate, together with succinate and acetate in molar proportions (for each 7 moles of glucose) between 0 and 2.

Heteropolysaccharides of this type are generated by a variety of micro-organisms, including those of the genera Pseudomonas, Rhizobium, Alcaligenes and Agrobacterium. Particularly suitable organisms include *Rhizobium meliloti, Alcaligenes faecalis* var. *myxodenes, Agrobacterium tumefaciens, Agrobacterium radiobacter, Agrobacterium rhizogenes,* and *Pseudomonas* sp. NCIB 11264, and also a novel strain of *Pseudomonas* species isolated by the Applicants from a sample of soil, and deposited at the National Collection of Industrial Bacteria, Torry Research Station, Aberdeen, under the Accession number 11592.

The heteropolysaccharide used in the process of this invention can be produced by cultivating the chosen micro-organism in an aqueous nutrient medium until substantial quantities of heteropolysaccharide are elaborated. The aqueous nutrient medium will normally contain an assimilable source of carbon and nitrogen, together with smaller amounts of magnesium, calcium, iron, phosphorus and other inorganic ions. The sources of nitrogen and carbon are suitably an ammonium salt and a carbohydrate respectively, the latter conveniently being glucose, and preferably being employed in a concentration between 0.1 and 10% by weight, normally 1—2% w/v. The temperature of cultivation and time required to generate an acceptable yield of heteropolysaccharide naturally vary according to the organism. In the case of *Pseudomonas* sp. NCIB 11592 the temperature is preferably between 20° and 35°C, and for a batch process the fermentation time is normally between 60—180 hours, generally from 80—100 hours.

When the fermentation is completed, the heteropolysaccharide may be recovered from the neat fermentation broth, or, more preferably, after the broth has been separated from the cells. This separation is conveniently carried out by adding a suitable diluent to give a concentration of polysaccharide of about 2000 ppm, and centrifuging the cells from the diluted broth. The clarified broth may then be treated by known techniques to recover the heteropolysaccharide from the fermentation broth. A convenient technique is that of solvent precipitation, in which the clarified fermentation broth is treated with a suitable inorganic salt, such as potassium chloride, and a water miscible solvent which does not react with the heteropolysaccharide and in which the product is insoluble. The product is thus precipitated and may be recovered by accepted and known techniques and dried. Typical organic solvents which may be used are straight or branched chain lower alkanols, e.g. methanol, ethanol and isopropanol. The preferred solvent is isopropanol, when the volume added is normally 1.5 times the volume of clarified fermentation beer.

However, it is not always essential to separate out the pure heteropolysaccharide, and a convenient process variant is to establish a steady state fermentation in which nutrient medium is continuously fed to the micro-organism, polysaccharide-containing nutrient medium is continuously withdrawn from the system and, after any necessary clarification, concentration adjustment and/or incorporation of additional compounds, is injected into the well.

It is believed that the heteropolysaccharide used in the process of this invention effectively comprises an octasaccharide repeating unit based on 7 D-glucose residues and 1 D-galactose residue. The heteropolysaccharide also contains varying proportions of certain acid residues, namely pyruvate, succinate and acetate. The precise proportions of these molecular components vary according to the micro-organism from which the heteropolysaccharide is produced, and also the precise conditions under which the organism is cultivated. Also the proportions as determined experimentally will show some degree of variability between successive sets of measurements on the same material, arising in part from a lack of complete homogeneity in the material and hence a real variation between samples, and in part from the limits of accuracy of the analytical techniques employed. As indicated earlier, pyruvate is always present in the heteropolysaccharide, but acetate and succinate may occur only in small amounts or not at all. As explained later, treatment of the polysaccharide with alkali will remove any succinate and acetate originally present, but it will leave the pyruvate intact. Such a polysaccharide, wherein the acetate and succinate content is effectively zero, has acceptable physical/rheological properties and falls within the scope of this invention.

3

Polymers suitable for use in the fluid displacement process of this invention should possess certain physical/rheological characteristics. The most basic of these is the inherent viscosity of the polymer in aqueous solution, since this is necessary for the effectiveness of the displacement. In common with many other water-soluble polymers, aqueous solutions of the heteropolysaccharides used in this process are pseudoplastic, or shear-thinning, that is their viscosity reversibly decreases with an increase in shear rate, and therefore the viscosity criteria have to be related to the shear rate. For commercially effective results it is preferred that a 1000 ppm solution of the heteropolysaccharide in a medium brine solution (as hereinafter defined) should have a viscosity of at least 3, and more especially at least 10, mPa.s when determined at a shear rate of 7.5 s$^{-1}$ and a temperature of 25°C. Determinations of the viscosity under these conditions can conveniently be made with a Brookfield LVT or Contraves Low Shear viscometer or a Deer rheometer.

The shear-thinning, or "pseudoplastic", characteristics of these polymers are of particular benefit when applying the polymers as viscosifiers in aqueous solutions used as displacing-agents for displacing fluids (such as oil) in a permeable sub-surface formation to a production well (or a plurality of production wells) communicating with the formation. The oil is then recovered to the surface via the production well(s). In such enhanced water-drive oil recovery process of this invention, the aqueous viscous solutions have a considerably greater injectivity than that of a fluid having comparable displacement characteristics but Newtonian flow properties. However, injectivity can be adversely affected by the retention of the polymer in the pores of the permeable subsurface formation, leading to plugging of the pores with consequential loss of injectivity and impairment of oil recovery. Suitable filtration tests known per se may be carried out to assess the potential hazard of plugging a formation of a particular permeability when a particular batch of the microbial polysaccharide produced by the production method according to the present invention is to be injected thereinto, and it is found that such hazard is minimal.

Another feature of the polysaccharide solution of importance for improved fluid displacement in wells and/or subsurface formations is that both the viscosity and filterability characteristics should be maintained in the presence of ionic salts, since the aqueous solutions used in oil recovery treatments are normally saline, and also the oil-bearing formations themselves often contain significant salt concentrations. The polysaccharides used in this invention generally fulfil these varied requirements better than materials hitherto available.

Characterisation of *Pseudomonas* sp. NCIB 11592

*Pseudomonas* sp. NCIB 11592 has the morphological and physiological characteristics set out below. These were determined by standard test methods described in "Cowan and Steel's Manual for the Identification of Medical Bacteria", second edition (1974). Comparison of these characteristics with those listed in "Bergey's Manual of Determinative Bacteriology, Eighth Edition" indicates that the organism has many characteristics of the genus Pseudomonas but certain properties which are normally associated with Agrobacterium, though the production of a water-soluble pigment under certain conditions suggests that Pseudomonas is the more appropriate designation.

1. Physical characteristics
   (a) Shape — small rods, occurring singly or in pairs. 0.5—1.0 microns, ×1.5—2.5 microns.
   (b) Motility — motile with 1 or 2 polar flagella
   (c) Sporulation — no evidence of spore (or cyst) formation.
   (d) Gram stain — negative.

2. Cultural characteristics
   Nutrient agar plate. Colonies off-white, smooth glistening flat, circular and entire. Diameter 2—3 mm after 24 h. at 30°C.

3. Physiological characteristics
   (a) Catalase — positive
   (b) Oxidase — positive
   (c) Urease production — positive
   (d) Growth — aerobic
   — anaerobic with nitrate
   (e) Temperature relations — up to 37°C, optimum 30°C. No growth at 4°C or 41°C.
   (f) pH relations — optimum 6.5—7.5, range 4.5—9.0
   (g) Methyl red — negative
   (h) Voges-Proskauer — negative
   (i) Carbohydrate breakdown — oxidative
   (j) H$_2$S production — negative
   (k) Indole production — negative

4

(l)   Nitrate reduction                              —   positive, $N_2$ or $NH_3$ formed
(m)  Hydrolysis of
         gelatin                                     —   negative
         Tween (registered Trade Mark)               —   negative
         Casein                                      —   negative
         starch                                      —   negative
(n)   Litmus milk                                    —   reduced
(o)   Arginine dihydrolase
         (Thornely's test)                           —   negative
(p)   Arginine decarboxylase                         —   negative
         Lysine decarboxylase                        —   negative
         Ornithine decarboxylase                     —   negative
(q)   Pigmentation                                   —   Kings B medium—slight yellow
(r)   Utilisation of carbon sources—Grows on glucose, sucrose, fructose, succinate, serine, alanine, mannitol, lactate, and propylene glycol. Acid produced from glucose. No growth on citrate, malonate, phenylalanine, gluconate, ethanol or ethylene glycol.
(s)   Bernaerts and De Ley test                      —   positive

Preparation of heteropolysaccharide by cultivation of Pseudomonas sp. NCIB 11592

a) Batch process

The inoculum for a batch culture was initially grown from a single mucoid colony of *Pseudomonas* sp. NCIB 11592 from an agar plate, in a 250 ml. conical shake flask containing "Lab-lemco" broth (100 ml.) and 10 gl$^{-1}$ glucose, and incubated at 30°C for 24 hours on an orbital shaker. An aliquot (40 ml.) of this culture was then transferred aseptically to a 2-litre conical shake flask containing 1 litre of the medium defined below and was incubated for a further 30 hours at 30°C on an orbital shaker before inoculation into the fermenter.

Medium composition

| | |
|---|---|
| Glucose | 10 g/l |
| $Na_2HPO_4$ | 3.0 g/l |
| $KH_2PO_4$ | 3.0 g/l |
| $(NH_4)_2SO_4$ | 0.3 g/l |
| $MgSO_4 . 7H_2O$ | 0.2 g/l |
| $FeCl_3 . 6H_2O$ | 66.8 mg/l |
| $CaCl_2 . 2H_2O$ | 14.7 mg/l |
| $ZnSO_4 . 7H_2O$ | 0.36 mg/l |
| $CuSO_4 . 5H_2O$ | 0.32 mg/l |
| $MnSO_4 . 4H_2O$ | 0.30 mg/l |
| $CoCl_2 . 6H_2O$ | 0.36 mg/l |
| $H_3BO_3$ | 0.20 mg/l |
| $Na_2MoO_4 . 2H_2O$ | 0.60 mg/l |

Fermentation was initiated by the addition of the inoculum into a Chemap LF-7 fermentation vessel containing 3/l of growth medium to give a final working volume of 4/l. The culture temperature was maintained at 28°C±0.2°C and the pH controlled at 6.80 by the automatic addition of a 1N potassium hydroxide+1N sodium hydroxide solution. Air was sparged into the fermenter at a rate of 0.50 l. min$^{-1}$ and the culture agitated by 3×4-bladed Rushton turbine impeller at a speed of 1000 rpm. Gas transfer under these conditions was sufficient to maintain the culture dissolved oxygen tension in the region of 120 to 140 mm. Hg.

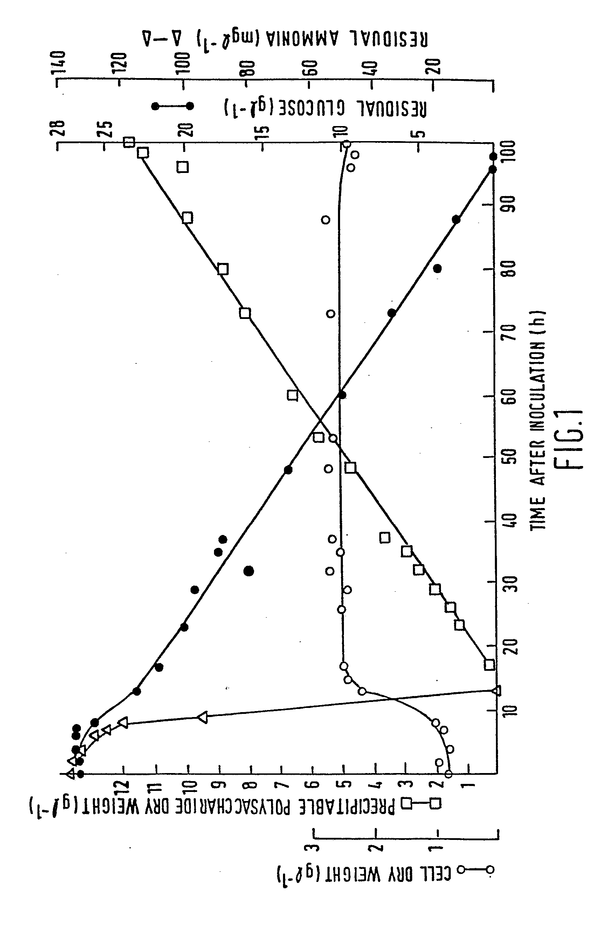

Culture broth samples (20 ml.) were taken at regular intervals throughout the fermentation and analysed for cell and expopolysaccharide concentration, residual medium glucose and residual medium ammonium ion. The progress of this culture, and the generation of heteropolysaccharide are summarised graphically in Figure 1.

When the fermentation was completed, the fermentation broth was separated from the cells by diluting with water to give a concentration of polysaccharide of about 2000 ppm and centrifuging the cells from the diluted broth, suitably with a Sharples Laboratory Super Centrifuge with liquid input running at 3 litre/h. The heteropolysaccharide is then recovered from the fermentation both by the addition of potassium chloride followed by isopropanol in an amount of 1.5 times the volume of clarified fermentation beer. The resultant gel like precipitate is removed from the spent fermentation beer, and is then pressed to remove residual isopropanol and dried under vacuum at 50°C. The dried material may then be ground to a fine powder.

b)   Continuous process

Following a procedure similar to that described above, a culture broth of *Pseudomonas* sp. NCIB

5

11592 was established in a 4 litre "Biotec" fermenter fitted with baffles and an impeller. This broth was then fed with two nutrient streams:—

| | | |
|---|---|---|
| Stream 1:— | $H_3PO_4$ | 1.088 g/l |
| | $MgSO_4 . 7H_2O$ | 0.493 g/l |
| | $CaCl_2 . 2H_2O$ | 0.147 g/l |
| | Glucose | 20 g/l |
| | Trace elements:— | as in medium defined above. |
| Stream 2:— | $(NH_4)_2SO_4$ | 21.14 g/l |

The volume of the broth was 2.6 litres, and nutrient streams 1 and 2 were continuously fed at rates of 149.2 and 15.4 ml/hour respectively, and the culture broth maintained at 28°C and pH 7.5 (by the use of 2.0N mixture of sodium hydroxide and potassium hydroxide). Culture medium was continuously withdrawn at an equivalent rate, and this outlet stream contained 3.7 g/l of polysaccharide (as estimated by viscosity) and 2.7 g/l of cells (determined by optical density and expressed as cell dry weight).

Determination of heteropolysaccharide chemical constitution

The heteropolysaccharide obtained from the above batch fermentation was hydrolysed with 0.25 M sulphuric acid at 95°C for 16 hours. Quantitative analysis by g.l.c. after conversion to the peracetylated aldononitrile derivatives, and by enzymatic methods revealed that the only neutral sugars present were D-glucose and D-galactose, the molar ratio of glucose:galactose being 7:0.96 and that pyruvate, acetate and succinate were also present in molar amounts (based on 7 moles of glucose) of 1.02; 0.11; and 1.11 respectively.

Titration of the deionized native polysaccharide gave the curve expected of a dibasic acid whereas titration of the alkali deacylated polymer indicated a single acidic group. Hence, the polysaccharide contains an alkali-stable acidic function, believed to be supplied by the pyruvate group linked as a ketal to the sugar residue, and also an alkali-labile acidic function believed to be derived from succinic acid in the form of its half ester. The acetyl content of the polymer has been found to vary somewhat between different batches, but is consistently less than equimolar with galactose. The succinyl content is normally approximately equimolar with galactose.

Methylation of the polysaccharide by the Hakomori procedure gave the methylated sugars shown in Table 1. A single g.l.c. system to resolve all the partially methylated sugars has not been found but the use of two systems has allowed a complete analysis. The individual methylated sugars were identified by g.c.—m.s. The presence of two distinct 2,4,6,tri-O-methyl hexose derivatives unequivocally shows that the galactose residue and one glucose residue are linked at the 3-position in the molecule. The remaining partially methylated sugars must all be derived from glucose residues. The methylation data are therefore best interpreted in terms of a polymer with an 8 sugar repeat unit (7 glucose+1 galactose). No tetramethyl derivative is evident but two 2,3 di-O-methyl glucose units occur per repeat unit. Hence, if the repeat unit is branched, the non-reducing terminal must consist of a pyruvylated glucose residue 4,6-O-(1-carboxyethylidene)-D-glucose which gives 2,3 di-O-methyl-glucose on methylation. The second 2,3 di-O-methyl glucose residue would be derived from the branching point sugar of the polysaccharide.

## TABLE 1

| Methylated sugar[+] | Column I | Mole ratios column II | Overall mole ratios |
|---|---|---|---|
| 2, 4, 6 Glucose | 2.08 | 2.06 | 2 |
| 2, 4, 6 Galactose ⎫ | 3.04 ⎫ | 1.00 | 1 |
| 2, 3, 6 Glucose ⎭ | | 2.91 | 2 |
| 2, 3, 4 Glucose | 1.00 ⎭ | | 1 |
| 2, 3, Glucose | 1.96 | 1.93 | 2 |

Column I=5% SE 30 on Supelcoport 100/120; 175°C; $N_2$ at 50 ml/min.
Column II=3% NPGS on Chromosorb W.A.W.; 180°C; $N_2$ at 50 ml/min+2,4,6 Glucose representing 2,4,6-tri-O-methyl glucose, etc.

In order to evaluate the extent of the variability in polysaccharide composition as determined on the product from a specific microorganism strain, several samples of polysaccharide were prepared from Pseudomonas NCIB 11592, and the product analysed enzymically after hydrolysis in 0.25M sulphuric acid at 95°C for 16 hours. The results are shown in Table 2 below:—

**0 040 445**

TABLE 2

| Polymer sample | Glucose | Galactose | Mole ratios pyruvate | Acetate | Succinate |
|---|---|---|---|---|---|
| A* | 7 | 0.96 | 1.02 | 0.10 | 1.12 |
| B* | 7 | 0.97 | 1.08 | 0.08 | 1.22 |
| C** | 7 | 0.98 | 0.92 | 0.01 | 1.07 |
| D** | 7 | 0.95 | 1.05 | 0.19 | 1.25 |

\* polysaccharide produced in batch culture
\*\* polysaccharide produced by continuous reactor.

A further detailed study was made on 6 batches of product from *Pseudomonas* NCIB 11592, hydrolysed in 0.5M sulphuric acid at 95°C for 16 hours. The sugar analyses were performed as before, and the acids were analysed by high pressure liquid chromatography on a 30 cm. "Bio-rad" HPX 87 column at 30°C with detection by UV absorption at 206 nm. The number of samples analysed from each batch was 3—5, and each sample was separately hydrolysed. The variation between the samples in each batch (representing the variation resulting from sampling, and in the actual analyses) was statistically analysed to give the standard error of the mean for each batch. Of the various analytical techniques, that for the determination of galactose is the least precise, the standard deviation being about 0.05.

The results are shown in Table 3 below.

TABLE 3

| Batch no. | Glucose | Mole ratio (based on Glucose as 7) Galactose | Pyruvate | Succinate |
|---|---|---|---|---|
| 1 | 7 | 0.99±.01 | 1.01 | 0.92 |
| 2 | 7 | 1.16±.02 | 0.77 | 1.02 |
| 4 | 7 | 1.06±.02 | 0.998±.005 | 1.145±.005 |
| 5 | 7 | 1.10±.01 | 0.71±.01 | 0.87±.02 |
| 6 | 7 | 1.01±.02 | 1.04±.03 | 1.10±.04 |

In all cases, acetate was detected, but was present below the level which could be accurately measured (about 0.3 mole ratio).

Sugar analyses similar to those described at the beginning of this section were carried out on the polysaccharides produced by a number of known organisms, with the results shown in Table 4 below.

TABLE 4

| Organism | Sugar glucose | Galactose | Pyruvate | Acid residues Acetate | Succinate |
|---|---|---|---|---|---|
| *Pseudomonas* sp. NCIB 11264 | 7.0 | 0.94 | 0.91 | 0.09 | 0.67 |
| *Rhizobium meliloti* K.24 | 7.0 | 1.00 | 1.00 | 1.20 | 1.65 |
| *Rhizobium meliloti* DSM 30136 | 7.0 | 1.05 | 1.02 | 1.07 | 1.98 |
| *Agrobacterium radiobacter* NCIB 8149 | 7.0 | 1.05 | 0.98 | 0.35 | 0.74 |
| *Agrobacterium radiobacter* NCIB 9042 | 7.0 | 0.94 | 1.00 | 0.14 | 0.57 |
| *Agrobacterium tumfaciens* DSM 30208 | 7.0 | 0.97 | 0.89 | 0.16 | 0.51 |

It is apparent from these results that the polysaccharides produced by each organism (including the novel *Pseudomonas* sp. NCIB 11592) have essentially similar sugar and pyruvate composition. Accordingly, it is believed that they all have the same basic octasaccharide repeating unit, which has been fully elucidated in respect of the polymer derived from a strain of *Rhizobium meliloti*—see J.Am.Chem.Soc. *99* (1977) 3812—3815, and all the polysaccharides having this composition are potentially suitable for use in the process of this invention.

7

Determination of heteropolysaccharide rheological properties

a)  Viscosity

The viscosity/shear properties were determined for the polysaccharides produced by a number of the organisms described above, and the results are set out in Table 5 below. In all cases the results refer to a 1000 ppm solution of the polysaccharide in medium salinity brine (as defined below), and were determined according to the procedures hereinbefore described.

TABLE 5

| Micro organism used | | Viscosity (mPa.s) | | |
|---|---|---|---|---|
| Shear rate (s$^{-1}$) | 11.5 | 23 | 46 | 115 |
| Pseudomonas sp. NCIB 11592 | 60 | 36 | 22 | 12 |
| Pseudomonas sp. NCIB 11264 | 40 | 28 | 21 | 12 |
| Rhizobium meliloti K.24 | 26 | 20 | 14 | 9.0 |
| Agrobacterium radiobacter NCIB 8149 | 17 | 15 | 12 | 9.0 |
| Deacylated product of NCIB 11592 | 14 | 13 | 12 | 10.4 |

The influence of salinity on viscosity was also evaluated using the polysaccharide from Pseudomonas sp. NCIB 11592 in a 1000 ppm aqueous solution containing different levels of sodium chloride concentration. All determinations were carried out at 25°C and a shear rate of 23 s$^{-1}$, and the results are shown in Table 6 below.

TABLE 6

| % Na Cl | 0 | 0.05 | 0.5 | 5.0 | 10.0 | 20.0 | 30.0 |
|---|---|---|---|---|---|---|---|
| Viscosity | 37.0 | 37.0 | 36.5 | 36.0 | 38.0 | 39.0 | 42.0 |

b)  Filterability

The potential hazard of formation plugging is assessed by an empirical filtration test in which the passage of a given volume of heteropolysaccharide solution through defined filter(s) is measured as a function of time. Generally, the solutions filter easily for so long as at least part of the filter is open to flow, but as soon as the filter pores become plugged the filtration rate declines rapidly. It has been established empirically that the filtration behaviour can be satisfactorily evaluated using filters of 47 mm diameter with a pressure difference of 1.0 bar and a sample volume of 100 ml of solution. Suitable filters are of cellulose ester type (e.g. "Millipore" (registered Trade Mark)) having pore sizes ranging from 0.45 to 5.0 microns, e.g. 0.45, 0.8, 1.2 or 5.0 microns. The performance is expressed by the time needed to pass the 100 ml through the filter. In order to determine the effect of ionic salts on the filterability characteristics, the filtration test is carried out at three levels of salinity, as follows:—

| Low Salinity contains | 0.5% NaCl, 0.05%, CaCl$_2$.2H$_2$O |
|---|---|
| Medium Salinity contains | 3% NaCl and 0.3% CaCl$_2$.2H$_2$O |
| High Salinity contains | 10% NaCl and 1% CaCl$_2$.2H$_2$O |

Solutions of 600 ppm of heteropolysaccharide are made up in each of these low, medium and high salinity brines and are evaluated for filterability as described above.

Determination of the filtration characteristics of the heteropolysaccharide produced from Pseudomonas sp NCIB 11592 demonstrated that at all levels of salinity the solutions passed in less than 3 minutes through filters of pore sizes 0.8 $\mu$; 1.2 $\mu$ and 5.0 $\mu$. In all cases it was found that the passage of the solutions through the filters did not lead to any loss in inherent viscosity of the polymer solution. Using the smallest, 0.45 $\mu$, filter pore size, the filtration times were determined for the polysaccharides produced by a number of the organisms described above, and the results are set out in Table 7 below. For Pseudomonas sp NCIB 11592 the test was carried out using a 600 ppm solution of the polymer; for the polymer produced from the other organisms a 1000 ppm solution was used.

TABLE 7

| Micro organism | Filtration time (seconds) | | |
|---|---|---|---|
| | Low saline | Medium saline | High saline |
| Pseudomonas sp NCIB 11592 | 175 | 245 | 360 |
| Pseudomonas sp NCIB 11264 | 1200 | 210 | 260 |
| Rhizobium meliloti K-24 | 426 | 49 | 208 |
| Agrobacterium radiobacter NCIB 8149 | 105 | 88 | 108 |

c)   Shear stability

An estimation of the shear stability of the polymer produced by *Pseudomonas* sp NCIB 11592 was made according to the following technique, and was compared with the results obtained from the known viscosifiers for oil operations, Kelzan XC and Pusher 700.

A Sorval Omnimixer was set up using the 400 ml container cooled in ice/water. About 175 ml of c. 1000 ppm polymer solution in distilled water was added and the mixer run at setting 4 (11500 rpm). An initial sample was taken, and samples taken at intervals of 30 s, 30 s, 1 min, 2 min, 4 min and 8 min, giving cumulative shear times of 30 s, 1 min, 2 min, 4 min, 8 min and 16 min respectively. Samples were taken (=4 ml) with a Pasteur pipette, and their viscosities (mPa.s) at 23 $S^{-1}$ shear rate determined. Viscosities of the extreme samples from the Kelzan experiment measured after 1 day showed no measurable recovery in viscosity.

The results are tabulated in Table 8 below.

TABLE 8

| Time (min.) | Kelzan XC | | Pusher 700 | | *Pseudomonas* NCIB 11592 polymer | |
|---|---|---|---|---|---|---|
| | Viscosity | % Initial viscosity | Viscosity | % Initial viscosity | Viscosity | % Initial viscosity |
| 0 | 24.3 | 100.0 | 38.0 | 100.0 | 37.3 | 100.0 |
| 0.5 | 23.7 | 97.5 | 28.0 | 73.7 | 35.7 | 95.7 |
| 1 | 22.9 | 94.2 | 23.0 | 60.5 | 36.8 | 98.7 |
| 2 | 22.4 | 92.9 | 17.2 | 45.3 | 35.6 | 95.4 |
| 4 | 20.9 | 86.0 | 12.4 | 32.6 | 35.0 | 93.8 |
| 8 | 18.6 | 76.5 | 9.2 | 24.2 | 33.6 | 90.1 |
| 16 | 16.0 | 65.8 | 6.6 | 17.4 | 30.8 | 82.6 |

The high resistance against salt and shear, and the low plugging properties render the present mircobially produced polysaccharide extremely useful as a viscosifier in processes wherein aqueous solutions are used for displacing fluids in wells and/or subsurface formations communicating with the wells. When applying the viscosifier in a process wherein a viscous aqueous displacement solution is used for displacing oil or other hydrocarboneous mixtures in a subsurface formation towards a production well (or production wells), the increased viscosity of the displacement fluid will be effective to minimize fingering of fluid through the oil present in the formation. The shear resistance and the salt resistance of the present polysaccharide will enable its desirable physical properties to be retained over the period that it is being displaced through the formation in the oil recovery process.

The process according to the present invention may be used not only for displacing oil, etc. in oil reservoirs to production wells, but also for cleaning a well by displacing the fluid originally present in the well. Such a process then forms part of a well completion or work-over operation. Cleaning the well is imperative after cementing the casing and prior to initiating production in the well. A viscous brine is used for this purpose, and the heteropolysaccharide herein defined is found to be extremely suitable as a viscosifier since it has, as already explained above, an excellent resistance against salt as well as low plugging properties. The salt resistance allows the use of brine as cleaning fluid which is attractive in view of the increased density of this fluid with respect to water. Further, there is no hazard of plugging the formation when part of the viscous brine enters the pore space of the formation at the level where the formation communicates with the well.

For cleaning the well, the viscous brine is injected into the well via a conduit extending into the well and thereafter returned to the surface via the annulus around the conduit. The fluid originally present in the well is displaced by the circulating viscous brine, and undesirable solids present in the well are simultaneously lifted from the well. The solids are removed from the circulating viscous brine by passing the brine through a filter at the surface, and since the viscous brine has low plugging properties, cleaning of the filter is only required when the amount of solids deposited thereon hampers the filtration rate. When the well has been cleaned, the viscous brine is temporarily left in the well over the period that further completion operations are carried out in the well. Such operations are known per se and need not be described here in detail. Apart from being suitable in production wells, the present process may also be included in completion operations carried out in injection wells, those are wells via which displacement fluids are passed into a subsurface formation.

The present process may also find application as part of a work-over operation carried out in either a production well or an injection well that has been in use for some time but needs to be repaired. The well is then cleaned by displacing the fluid present therein by means of the present process, and the displacement fluid is left in the well during the period that repair operations are carried out in the well. To prevent a blow-out from the well, the cleaning fluid may be weighted by suitable weighting agents known per se for increasing the density of work-over fluids.

In summary, the present process is suitable for use in carrying out any operations in wells and/or formations wherein a fluid is being displaced through the well and/or pore space of the formation. Apart from the application of the present process in the above described work-over-, completion, oil recovery-, and fracturing-operations, the process may also be used in any other type of operations wherein a viscous aqueous solution is pumped down a well, such as for the transport of sand particles, etc., down the well and into the formation, thereby displacing the fluid originally present in the well and/or formation. The use of the present microbially produced polysaccharide will prevent the formation becoming plugged by the viscous aqueous solution on entering the pore space of the formation. Also, the required viscosity for carrying out the process will not decrease when the viscosifier is in contact with salt solutions or salt layers.

## Claims

1. A process for displacing a fluid through a permeable subsurface formation communicating with a well, by injecting into the formation via said well an aqueous solution, characterised in that the solution contains as viscosifier a heteropolysaccharide comprising glucose and, for each 7 moles of glucose, 0.9—1.2 moles of galactose, and 0.65 to 1.1 moles of pyruvate, together with succinate and acetate in molar proportions (based on 7 moles of glucose) between 0 and 2.

2. Process as claimed in claim 1, wherein an aqueous solution of the heteropolysaccharide has a viscosity (as measured on a 1000 ppm solution in medium brine at a shear rate of 7.5 $s^{-1}$ and a temperature of 25°C) of at least 3.0 mPa.s.

3. Process as claimed in claim 2, wherein the aqueous solution has a viscosity of at least 10 mPa.s.

4. Process as claimed in claim 1, 2 or 3, wherein the polysaccharide is obtained by cultivation of a slime forming species of Pseudomonas, Rhizobium, Alcaligenes or Agrobacterium.

5. Process as claimed in claim 4, wherein the polysaccharide is obtained by cultivation of *Rhizobium meliloti, Rhizobium trifolii, Alcaligenes faecalis* var. *myxogenes, Agrobacterium tumefaciens, Agrobacterium radiobacter, Agrobacterium rhizogenes, Pseudomonas* sp. NCIB 11264, *Pseudomonas* sp. NCIB 11592, or mutants thereof, and, if required, subsequent alkaline deacylation of the microbial product.

6. Process according to any one of the preceding claims, wherein the aqueous solution that is injected into the well is passed into the formation for displacing at least part of the fluid present therein to a production well and recovering the fluid therefrom.

7. Process according to any one of the claims 1—5, wherein the aqueous solution that is injected into the well is for at least the major part of the injection allowed to circulate through the well via a conduit extending into the well and the annulus around the conduit for cleaning the well from the fluid contents originally present therein.

8. Process according to claim 7, wherein the aqueous solution after having been circulated once through the well, is filtered and re-injected into the well.

9. Process according to claims 7 and 8, wherein after the well has been cleaned, the aqueous solution is left stationary in the well during completion or work-over operations that are subsequently carried out in the well.

10. Process according to any one of claims 1—5, wherein the major part of the aqueous solution that has been injected into the well is passed into a fracture communicating with the well and extending into the formation.

11. A process for the production of a polysaccharide suitable for use in the process of claim 4, which comprises cultivating *Pseudomonas* sp. NCIB 11592, or mutants thereof, under aerobic conditions in an aqueous nutrient medium and recovering the polysaccharide from the polysaccharide-containing medium.

12. The process as claimed in claim 11, wherein the cultivation is carried out at a temperature between 20° and 35°C and a pH between 6.0 and 9.0.

13. A polysaccharide when produced by a process as claimed in claim 11 or 12.

## Patentansprüche

1. Verfahren zur Verdrängung einer Flüssigkeit aus einer durchlässigen Formation unterhalb der Oberfläche, die mit einem Bohrloch in Verbindung steht, durch Einspritzen einer wäßrigen Lösung in diese formation über das Bohrloch, dadurch gekennzeichnet, daß die Lösung als Viskositätserhöher ein Heteropolysaccharid enthält, umfassend Glucose und auf jeweils 7 mol Glucose 0,9 bis 1,2 mol Galactose und 0,65 bis 1,1 mol Pyruvat zusammen mit Succinat und Acetat in Molverhältnissen (bezogen auf 7 mol Glucose) zwischen 0 und 2.

2. Verfahren nach Anspruch 1, wobei die wäßrige Lösung des Heteropolysaccharids eine Viskosität (gemessen an einer 1000 ppm Lösung in mittlerer Salzlösuhg bei einer Scherrate von 7,5 $s^{-1}$ und einer Temperatur von 25°C) von mindestens 3,0 mPa.s besitzt.

3. Verfahren nach Anspruch 2, wobei die wäßrige Lösung eine Viskosität von mindestens 10 mPa.s besitzt.

4. Verfahren nach Anspruch 1, 2 oder 3, wobei das Polysaccharid erhalten worden ist durch Züchtung einer Schleim bildenden Art von Pseudomonas, Rhizobium, Alcaligenes oder Agrobacterium.

5. Verfahren nach Anspruch 4, wobei das Polysaccharid erhalten worden ist durch Züchtung von Rhizobium meliloti, Rhizobium trifolii, Alcaligenes faecalis, var. myxogenes, Agrobacterium tumefaciens, Agrobacterium radiobacter, Agrobacterium rhizogenes, Pseudomonas sp. NCIB 11264, Pseudomonas sp. NCIB 11592 oder Mutanten davon und wenn erforderlich anschließende alkalische Deacylierung des mirkobiologischen Produktes.

6. Verfahren nach einem der vorangehenden Ansprüche, wobei die wäßrige Lösung, die in das Bohrloch eingespritzt wird, in die Formation geleitet wird, um zumindest einen Teil der darin enthaltenen Flüssigkeit zu einem Förderbohrloch zu treiben und die Flüssigkeit daraus zu gewinnen.

7. Verfahren nach einem der Ansprüche 1 bis 5, wobei die wäßrige Lösung, die in das Bohrloch eingespritzt wird, zumindest zum größten Teil über eine Leitung durch das Bohrloch zirkulieren kann, die sich in das Bohrloch erstreckt und den Umkreis der Leitung, um das Bohrloch von den ursprünglich darin enthaltenen Flüssigkeiten zu reinigen (freizuhalten).

8. Verfahren nach Anspruch 7, wobei die wäßrige Lösung, nachdem sie einmal durch das Bohrloch gelaufen ist, filtriert und erneut in das Bohrloch injiziert wird.

9. Verfahren nach Anspruch 7 oder 8, wobei nach der Reinigung des Bohrloches die wäßrige Lösung während der weiteren Arbeiten, die anschließend in dem Bohrloch ausgeführt werden, in diesem stationär gehalten wird.

10. Verfahren nach einem der Ansprüche 1 bis 5, wobei der größere Teil der wäßrigen Lösung, die in das Bohrloch eingespritzt worden ist, in einen Bruch geleitet wird, der mit dem Bohrloch in Verbindung steht und sich in die Formation (das Gebirge) erstreckt.

11. Verfahren zur Herstellung eines Polysaccharids, das zur Anwendung bei dem Verfahren nach Anspruch 4 geeignet ist, umfassend die Züchtung von Pseudomonas sp. NCIB 11592 oder Mutanten davon unter aeroben Bedingungen in einem wäßrigen Nährmedium und Gewinnung des Polysaccharids aus dem Polsaccharid-haltigen Medium.

12. Verfahren nach Anspruch 11, wobei die Züchtung bei einer Temperatur zwischen 20 und 35°C und einem pH-Wert zwischen 6,0 und 9,0 durchgeführt wird.

13. Polysaccharid, das gebildet worden ist nach einem Verfahren wie in Anspruch 11 oder 12 beansprucht.


**Revendications**

1. Un procédé pour déplacer un fluide à travers une formation souterraine perméable communiquant avec un puits, en injectant dans la formation par le puits une solution aqueuse, caractérisé en ce que la solution contient comme agent de viscosité un hétéropolysaccharide comprenant du glucose et, pour 7 moles de glucose, 0,9 à 1,2 mole se galactose et 0,65 à 1,1 mole de pyruvate, ainsi que du succinate et de l'acétate dans des proportions comprises entre 0 et 2 moles pour 7 moles de glucose.

2. Procédé selon la revendication 1, dans lequel une solution aqueuse de l'hétéropolysaccharide a une viscosité (telle que mesurée sur une solution à 1000 ppm dans de la saumure moyenne à un taux de cisaillement de 7,5 $s^{-1}$ et à une température de 25°C) d'au moins 3,0 mPa.s.

3. Procédé selon la revendication 2, dans lequel la solution aqueuse a une viscosité d'au moins 10 mPa.s.

4. Procédé selon la revendication 1, 2 ou 3, dans lequel le polysaccharide est obtenu per culture d'une espèce de Pseudomonas, Rhizobium, Alcaligenes ou Agrobacterium formant une substance visqueuse.

5. Procédé selon la revendication 4, dans lequel le polysaccharide est obtenu per culture de *Rhizobium meliloti, Rhizobium trifolii, Alcaligenes faecalis* var. *myxogenes, Agrobacterium tumefaciens, Agrobacterium radiobacter, Agrobacterium rhizogenes, Pseudomonas* sp. NCIB 11264, *Pseudomonas* sp. NCIB 11592 ou leurs mutants et, si nécessaire, désacylation alcaline ultérieure du produit microbien.

6. Procédé selon l'une quelconque des revendications précédentes, dans lequel la solution aqueuse qui est injectée dans le puits est passée dans la formation pour déplacer au moins une partie du fluide qui y est présent vers un puits de production et pour recueil du fluide.

7. Procédé selon l'une quelconque des revendications 1 à 5, dans lequel on permet à la solution aqueuse qui est injectée dans le puits, pendant au moins la majeure partie de l'injection, de circuler à travers le puits par un conduit s'étendant dans le puits et par l'espace annulaire autour du conduit pour nettoyer le puits en chassant le fluide initialement présent.

8. Procédé selon la revendication 7, dans lequel la solution aqueuse, après circulation une fois à travers le puits, est filtrée et réinjectée dans le puits.

9. Procédé selon les revendications 7 et 8, dans lequel après que le puits a été nettoyé, la solution aqueuse est laissée en repos dans le puits durant des opérations de complétion ou de reconditionnement qui sont effectuées ensuite dans le puits.

10. Procédé selon l'une quelconque des revendications 1 à 5, dans lequel la majeure partie de la

# 0 040 445

solution aqueuse qui a été injectée dans le puits est passée dans une fracture communiquant avec le puits et s'étendant dans la formation.

11. Un procédé pour la production d'un polysaccharide utilisable dans le procédé selon la revendication 4, qui comprend la culture de *Pseudomonas* sp. NCIB 11592, ou de ses mutants, dans des conditions aérobies dans un milieu nutritif aqueux et le recueil du polysaccharide à partir du milieu contentant le polysaccharide.

12. Le procédé selon la revendication 11, dans lequel la culture est conduite à une température comprise entre 20°C et 35°C et à un pH compris entre 6,0 et 9,0.

13. Un polysaccharide quand il est produit par un procédé selon la revendication 11 ou 12.

FIG.1